# EUROPEAN PATENT APPLICATION

(11) **EP 4 424 317 A1**
(43) Date of publication of application: **04.09.2024**
(21) Application number: 22886104.3
(22) Date of filing: 28.10.2022
(51) Int. Cl.: A61K 35/57, A61P 1/00

(54) **MEDICINE FOR PREVENTING OR TREATING ENTERITIS AND INTESTINAL CANCER**

(30) Priority: 29.10.2021 CN 202111268772
(71) Applicant: Anhui Hygeiancells Biomedical Co. Ltd, Huangshan City, Anhui 245021 (CN)
(72) Inventor: QIAN, Jin, Huangshan City, Anhui 245021 (CN); LI, Shiyan, Huangshan City, Anhui 245021 (CN); WANG, Rong, Huangshan City, Anhui 245021 (CN)
(74) Representative: Biggi, Cristina
(86) International application number: PCT/CN2022/128160
(87) International publication number: WO 2023/072229

(57) **Abstract**

Provided is a medicine for preventing or treating enteritis and intestinal cancer. Specifically, the present invention relates to a use of amniotic fluid in the preparation of a medicine for treating or preventing enteritis in a subject, a use in the preparation of a medicine for preventing intestinal cancer in a subject, and a use in the preparation of a medicine for activating Nrf2 and inhibiting NF-κB to reduce inflammation and oxidative damage caused by damage to the intestinal mucosa barrier of a subject; the amniotic fluid comes from eggs at embryo ages of 5-12 days, eggs from birds other than chickens at the development stage corresponding to the development stage of chickens at said embryo age, embryos of rodents at a gestational age of 8-14 days, or embryos of non-human mammals other than rodents at the development stage corresponding to the development stage of rodents at a gestational age of 8-14 days.

## Description

### FIELD OF THE INVENTION

The present invention relates to medicines for preventing or treating enteritis and intestinal cancer.

### BACKGROUND OF THE INVENTION

Enteritis is intestinal inflammation caused by bacteria, viruses, fungi, parasites, or unknown factors, including enteritis of small intestine and colitis. Main clinical manifestations of enteritis include abdominal pain, diarrhea, loose watery stools or mucus-pus-bloody stools. Based on the course of disease, enteritis is divided into two categories: acute and chronic enteritis. The course of chronic enteritis is generally more than two months. Clinically, common chronic enteritis includes chronic bacillary dysentery, chronic amoebic dysentery, schistosomiasis, non-specific ulcerative colitis and regional enteritis, etc.

Ulcerative colitis (UC) is a chronic, recurrent inflammatory disease that affects the colon and rectum; it is characterized by body weight loss (BW), diarrhea, colonic/rectal inflammation, hematochezia, and ulcers. Currently, the pathogenesis of UC is still unclear. However, it is believed that environmental and genetic factors, oxidative stress, colonic inflammation, intestinal microbiota imbalance, and dysfunction of the mucosal immune response are related to the disease progression. The colonic mucus layer forms an important barrier between the external environment and the host's internal environment, which can regulate the interaction between intestinal flora and immunity. Presently, medicines for treatment of UC, such as corticosteroids, thiopurines and aminosalicylic acids, are relatively ineffective and often lead to serious adverse events. Therefore, it is necessary to develop a more effective and less toxic therapeutic drug.

Cellular oxidative stress involves a series of signaling that can contribute to the occurrence and progression of inflammatory diseases. Nuclear erythroid 2-related factor 2 (Nrf2) is the main transcription factor that can bind to genes containing antioxidant response elements (AREs) to activate antioxidant-related genes, such as NAD(P)H quinone oxidoreductase 1 (NQO1), heme oxygenase 1 (HO-1), etc. It is reported that Nrf2 can regulate the inflammatory response in UC and colitis-related intestinal cancer. In addition, in another study, Nrf2 activators are used to alleviate dextran sulfate sodium (DSS)-induced chronic and acute colitis. The Nrf2 signaling pathway can activate multiple signaling pathways and plays a crucial role in mediating inflammation and oxidative stress. Furthermore, Nrf2 can exert certain anti-inflammatory effects by inhibiting the production of pro-inflammatory cytokines IL-1β, IL-6 and TNF-α. The overexpression of pro-inflammatory cytokines is considered to be a prominent feature of reactive oxygen species (ROS)-induced inflammation in UC through the nuclear factor-κB (NFκB) pathway. Overall, targeting the Nrf2 and NPκB pathways simultaneously is an effective strategy for the treatment and prevention of UC.

### SUMMARY OF THE INVENTION

In a first aspect, the present invention provides use of amniotic fluid in the preparation of medicines for treating or preventing enteritis in a subject; wherein, the amniotic fluid is from eggs with an embryonic age of 5-12 days, preferably from eggs with an embryonic age of 6-11 days, more preferably from eggs with an embryonic age of 7-9 days, still more preferably from eggs with an embryonic age of 7-8 days, or from eggs of poultry other than chickens whose development stage corresponds to the development stage of eggs of the embryonic age; or from embryos of rodents with an embryonic age of 8-14 days, or from embryos of non-human mammals other than rodents whose development stage corresponds to the development stage of rodents with an embryonic age of 8-14 days.

In one or more embodiments, the enteritis includes but not limited to enteritis of small intestine and colitis.

In one or more embodiments, the enteritis is chronic enteritis, including chronic bacillary dysentery, chronic amoebic dysentery, schistosomiasis, non-specific ulcerative colitis and regional enteritis, etc.

In one or more embodiments, the enteritis is an inflammatory bowel disease, including, but not limited to, ulcerative colitis and Crohn's disease.

In a second aspect, the present invention provides use of amniotic fluid in the preparation of medicines for preventing intestinal cancer in a subject; wherein, the amniotic fluid is from eggs with an embryonic age of 5-12 days, preferably from eggs with an embryonic age of 6-11 days, more preferably from eggs with an embryonic age of 7-9 days, still more preferably from eggs with an embryonic age of 7-8 days, or from eggs of poultry other than chickens whose development stage corresponds to the development stage of eggs of the embryonic age; or from embryos of rodents with an embryonic age of 8-14 days, or from embryos of non-human mammals other than rodents whose development stage corresponds to the development stage of rodents with an embryonic age of 8-14 days.

In a third aspect, the present invention provides use of amniotic fluid in the preparation of drugs for, including but not limited to, activating Nrf2 and inhibiting NF-κB to alleviate inflammation and oxidative damage caused by destruction of the intestinal mucosal barrier in a subject; wherein, the amniotic fluid is from eggs with an embryonic age of 5-12 days, preferably from eggs with an embryonic age of 6-11 days, more preferably from eggs with an embryonic age of 7-9 days, still more preferably from eggs with an embryonic age of 7-8 days, or from eggs of poultry other than chickens whose development stage corresponds to the development stage of eggs of the embryonic age; or from embryos of rodents with an embryonic age of 8-14 days, or from embryos of non-human mammals other than rodents whose development stage corresponds to the development stage of rodents with an embryonic age of 8-14 days.

### BRIEF DESCRIPTION OF THE DRAWINGS

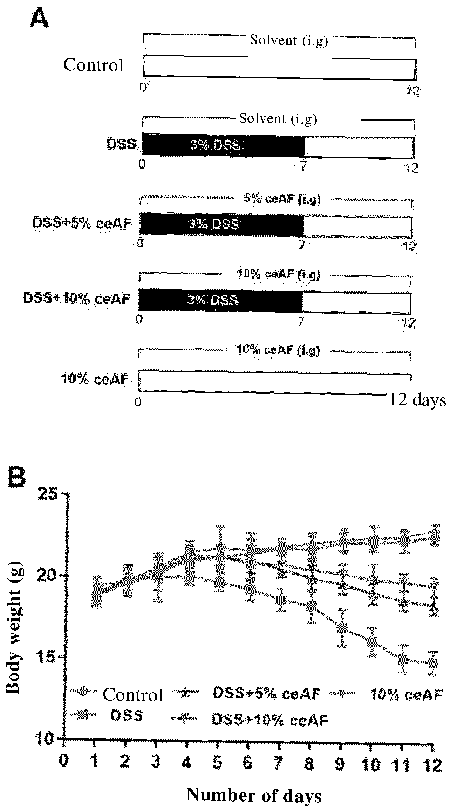

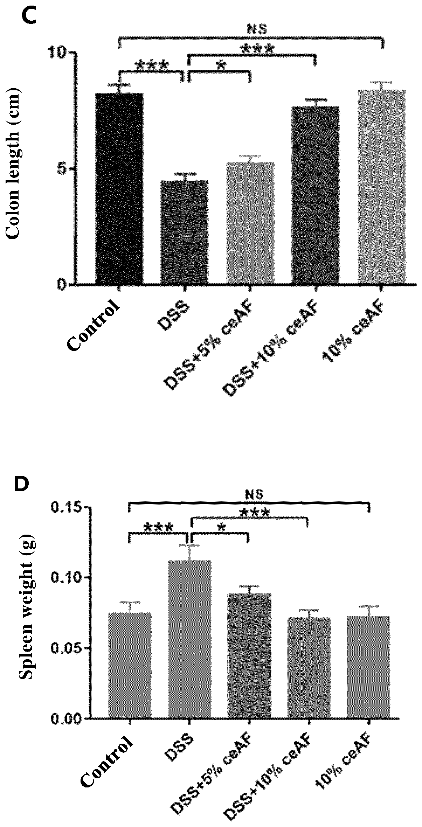

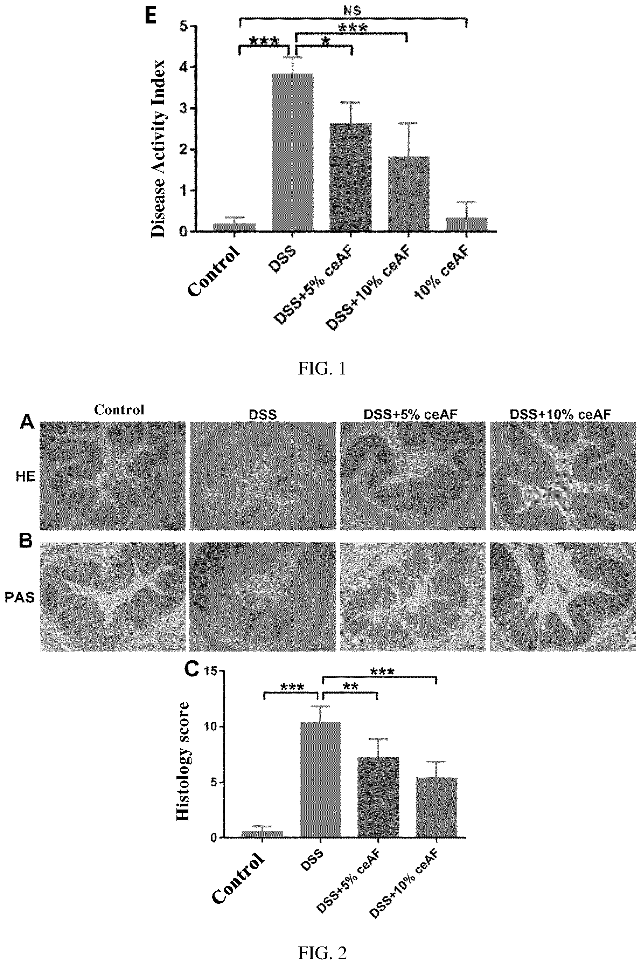

FIG.1: After mice are given sterile water with 3% DSS for 7 days and normal water for 7 days, changes in relevant parameters during the process: (A) chick early amniotic fluid (ceAF) improves morphological symptoms of DSS-induced acute colitis in mice, (B) body weights of mice; (c) colon length, (D) spleen weight and (E) disease activity index (DAI). Data are expressed as mean ± SD; *P<0.05, **P<0.01, ***P<0.001.
FIG.2: ceAF has a therapeutic effect on the colon structure and goblet cell abundance of mice with DSS-induced colitis. There is no obvious morphological damage or abnormality in main organs of mice. (A) H&E stained images and (C) histological scores of representative sections. Scale: 200 µm. (B) Representative images of PAS staining. Scale: 100 µm.

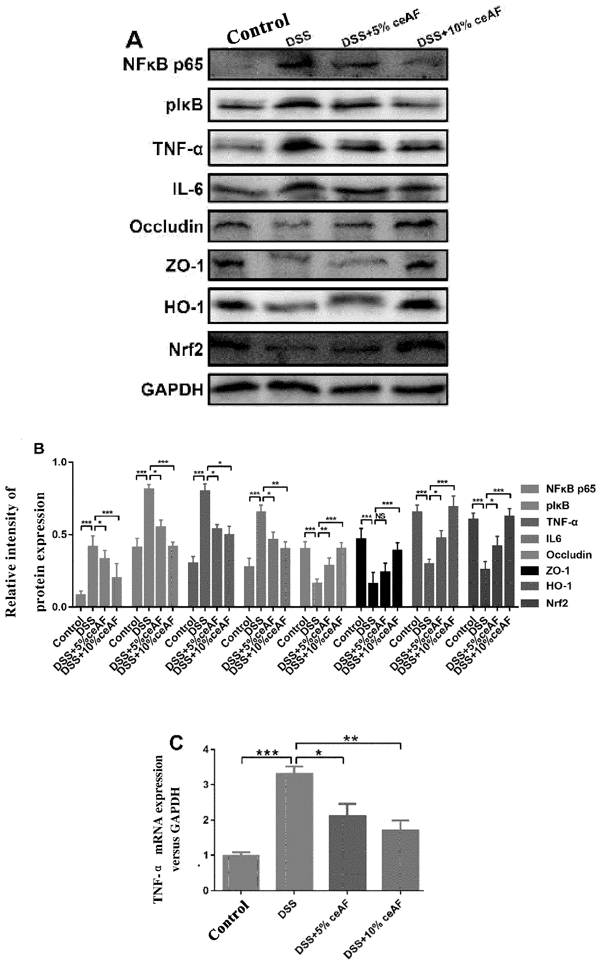

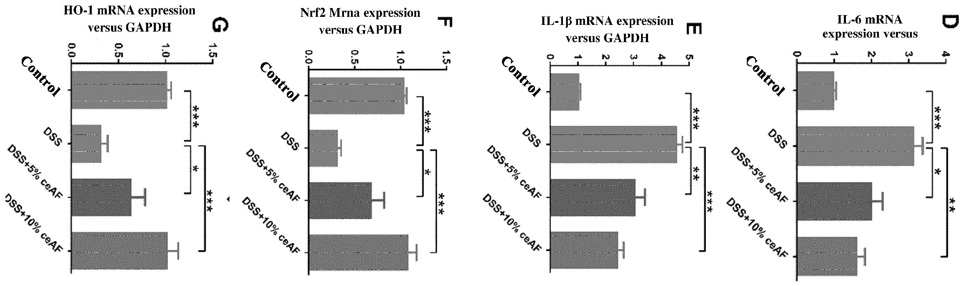

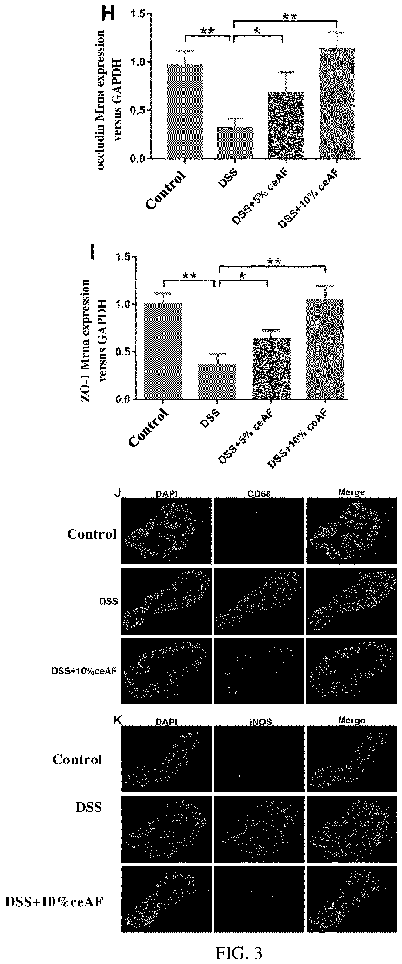

FIG.3: ceAF exhibits anti-inflammatory and antioxidant activities in DSS-induced colitis by regulating DSS-induced inflammatory responses and colonic barrier dysfunction. (A) Expressions of NPκB p65, pIκB, IL6, Nrf2, HO-1, ZO-1, and Occludin in colon tissues assessed by Western blotting, using GAPDH as the internal reference. (B) Relative expression intensity of NPκB p65, pIκB, IL6, Nrf2, HO-1, ZO-1, and Occludin after normalization using GAPDH. Determination of mRNA expression levels of (C) TNF-α, (D) IL-6, (E) IL-1β, (F) Nrf2, (G) HO-1, (H) Occludin, and (I) ZO-1 genes in colon tissues by real-time PCR. Confocal laser image of (J) CD68 and (K) iNOS labeling in M1 macrophages. Scale: 500 µm. Data are expressed as mean ± SD; *P<0.05, **P<0.01, ***P<0.001, n=10.

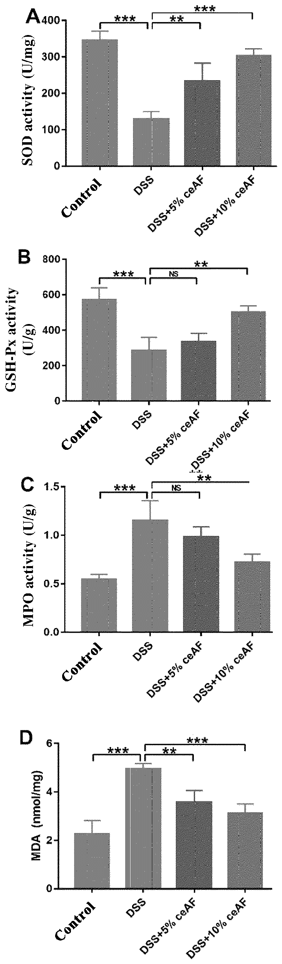

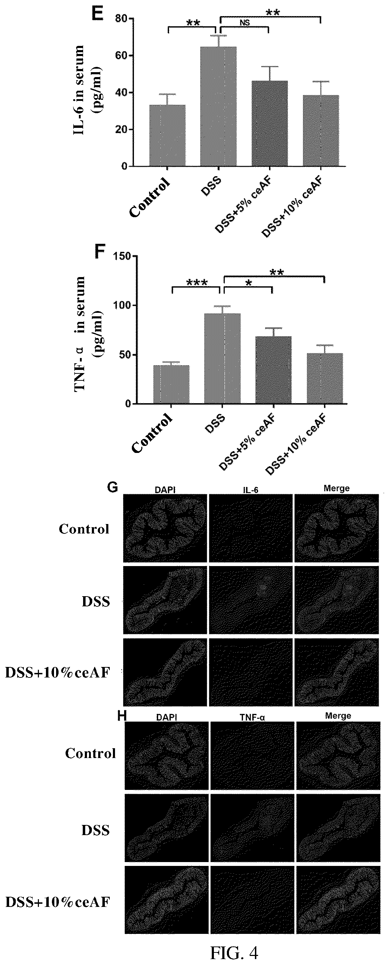

FIG.4: ceAF alleviates oxidative stress and inflammation in mice with DSS-induced colitis. The levels of (A) SOD, (B) GSH-Px, (C) MPO, and (D) MDA in colon tissues are detected by chemical chromatography. The levels of (E) IL-6 and (F) TNF-α in serum are detected by ELISA. (G, H) Immunostaining of colon tissue sections. All representative images are captured using an inverted fluorescence microscope. Data are expressed as mean ± SD. *P<0.05, **P<0.01 and ***P<0.001; ns: No significant difference, n = 10.

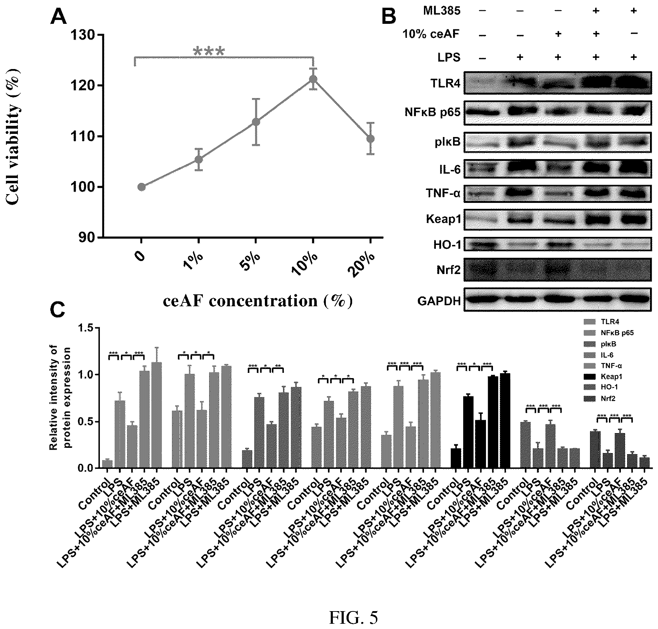

FIG.5: ceAF inhibits inflammation and oxidative stress in LPS-stimulated RAW264.7 cells by activating Nrf2 and inhibiting the NPκB pathway. (A) Viability of RAW 264.7 cells after treatment with different concentrations of ceAF. (B) Western blot of related proteins in the NPκB and Nrf2 signaling pathways after treatment with ceAF. The expressions of TLR4, NPκB p65, pIκB, IL6, TNF-α, Keap1, Nrf2 and HO-1 are detected, using GAPDH as the internal reference. (C) Densitometry quantification of the Western blot bands shown in (B). Data are expressed as mean ± SD; *P<0.05, **P<0.01, ***P<0.001.

### DETAILED DESCRIPTION

It should be appreciated that the aforementioned technical features and the technical features specifically described below (for example, embodiments) of the present invention can be combined with each other to constitute preferred technical solutions.

The inventor(s) have discovered that non-human animal amniotic fluid can activate Nrf2 and inhibit NF-κB, thereby reducing inflammation and oxidative damage caused by intestinal mucosal barrier damage; thus the non-human animal amniotic fluid can be used to treat or prevent enteritis and prevent intestinal cancer related to enteritis.

In the present invention, amniotic fluid can be derived from avian eggs and non-human mammals. Poultry eggs refer to eggs of poultry. Preferred poultry are domestic fowls, such as chickens, ducks and geese. Preferably, poultry eggs with an embryonic age of 5-20 days, preferably 6-15 days are used in the present invention. It should be understood that the appropriate embryonic ages for different eggs may not be the same. For example, when chicken eggs are used, preferably eggs with an embryonic age of 5-12 days, more preferably eggs with an embryonic age of 6-11 days, still more preferably eggs with an embryonic age of 7-9 days, and still more preferably eggs with an embryonic age of 7-8 days are used. When eggs from other poultry species are used, eggs whose development stage corresponds to the development stage of chicken eggs of the embryonic ages may be used. For example, when duck eggs are used, duck eggs with an embryonic age of 8-10 days, particularly 8-9 days, may be best.

Amniotic fluid of poultry eggs can be obtained using conventional methods. For example, the blunt end of an egg of corresponding embryonic age can be knocked to break the eggshell, and the eggshell can be peeled off to form a hole about 2 cm in diameter. Then the shell membrane and vitelline membrane are torn off carefully with tweezers, attention should be paid not to damage the amniotic membrane. The amniotic membrane and connected tissues surrounding the embryo are poured from the shell into a petri dish, and a syringe is used to pierce the amniotic membrane to extract amniotic fluid until the amniotic membrane is close to the embryo, thereby obtaining amniotic fluid used in the present invention.

In the present invention, amniotic fluid may also be derived from non-human mammals, especially rodents, for example, from mice. Other non-human mammals may be common domestic animals, such as cattle, sheep, dogs, cats, pigs, etc. In some embodiments, the amniotic fluid is derived from an embryo of a rodent with an embryonic age of 8-14 days, or from an embryo of a non-human mammal whose development stage corresponds to the development stage of the rodent with an embryonic age of 8-14 days. Amniotic fluid can be obtained by conventional methods. For example, surgical scissors are used to cut the abdominal cavity of a mouse whose pregnant period is 8-14 days to carefully remove and cut the uterus, and a syringe is used to pierce the amniotic membrane to extract amniotic fluid until the amniotic membrane is close to the embryo, thereby obtaining amniotic fluid used in the present invention.

It should be understood that when necessary, the amniotic fluid may be centrifuged to separate potential impurities, for example, egg yolk, to obtain pure amniotic fluid as much as possible. The supernatant obtained after centrifugation is the amniotic fluid used in the present invention. It should be understood that all steps to obtain amniotic fluid need to be performed under aseptic conditions; in addition, the "amniotic fluid" shown herein should be "pure" amniotic fluid, i.e., amniotic fluid separated from poultry eggs or non-human mammalian embryos that contains no other components in avian eggs or non-human mammalian embryos and is not contaminated by exogenous substances. Pure amniotic fluid may be stored in a refrigerator below -60°C and thawed before use.

The amniotic fluid described herein can be used as an active ingredient of a medicine for *in vivo* administration to a subject in need. For example, a subject in need thereof can be administered an effective amount of amniotic fluid described herein, or a pharmaceutical composition containing the amniotic fluid.

In the present invention, a subject may be an animal, for example a mammal, especially a human.

In the present invention, in terms of the disease site, enteritis includes enteritis of small intestine and colitis. Based on the course of disease, enteritis is divided into two categories: acute and chronic enteritis. Clinically, common chronic enteritis includes chronic bacillary dysentery, chronic amoebic dysentery, schistosomiasis, non-specific ulcerative colitis and regional enteritis, etc. In some embodiments, enteritis is inflammatory bowel disease (IBD). IBD is a special chronic intestinal inflammatory disease, including ulcerative colitis (UC) and Crohn's disease (CD), etc. UC is a chronic non-specific, non-infectious, inflammatory intestinal disease that mainly involves the colorectal mucosa and submucosa. The disease is characterized by continuous and diffuse distribution and is a continuous mucosal ulcer in the rectum and colon. It starts in the rectum and extends to different extents, up to cecum. UC is a common inflammatory bowel disease (IBD) and is associated with the occurrence of intestinal cancer. UC is mainly associated with immune abnormality and genetic mutations, while infection, diets, living habits, environmental factors, mental emotions, etc. are indispensable inducing factors.

Studies have shown that chronic ulcerative colitis can be complicated by intestinal cancer. The occurrence of intestinal cancer may be related to the chronic inflammatory stimulation of colon mucosa. It is generally believed that during the process of inflammatory proliferation, canceration occurs through the inflammatory polyp stage. Accordingly, in some embodiments, the present invention also relates to the use of the non-human animal amniotic fluid or pharmaceutical composition described herein for preventing intestinal cancer; the intestinal cancer is associated with the chronic inflammatory stimulation of colonic mucosa.

In particularly preferred embodiments of the present invention, amniotic fluid, especially the amniotic fluid from poultry eggs described herein, more preferably the amniotic fluid from chicken eggs is used for the treatment and prevention of UC, and for the prevention of intestinal cancer associated with the chronic inflammatory stimulation of colonic mucosa.

In some embodiments of the present invention, amniotic fluid, especially the amniotic fluid from poultry eggs described herein, more preferably the amniotic fluid from chicken eggs is used for one or more of the following uses: (1) retarding or preventing colon shortening in a subject, (2) inhibiting splenomegaly in a subject, (3) alleviating the progression of colitis, (4) mitigating the symptoms (for example, colon mucosal damage, loss of tissue structure, epithelial erosion, reduction in the number of glands, and inflammatory cell infiltration, etc.) of acute enteritis (for example, acute UC) in a subject, (5) increasing the abundance of goblet cells and improving goblet cell morphology in a subject to produce more mucus, and (6) downregulating the expressions of NF-κB p65, PIκB, IL-6 and TNF-α and upregulating the expressions of Occludin, ZO-1, Nrf2 and HO-1 in a subject. Preferably, the subject is a patient with enteritis. In some embodiments, the present invention relates to use of amniotic fluid, especially the amniotic fluid from poultry eggs described herein, more preferably the amniotic fluid from chicken eggs, in the preparation of one or more formulations for one or more of the following uses: (1) retarding or preventing colon shortening in a subject, (2) inhibiting splenomegaly in a subject, (3) alleviating the progression of colitis, (4) mitigating the symptoms (for example, colon mucosal damage, loss of tissue structure, epithelial erosion, reduction in the number of glands, and inflammatory cell infiltration, etc.) of acute enteritis (for example, acute UC) in a subject, (5) increasing the abundance of goblet cells and improving goblet cell morphology in a subject to produce more mucus, and (6) downregulating the expressions of NF-κB p65, PIκB, IL-6 and TNF-α and upregulating the expressions of Occludin, ZO-1, Nrf2 and HO-1 in a subject.

Accordingly, the present invention provides a method of reducing the severity of enteritis, or treating and preventing enteritis, or preventing intestinal cancer associated with chronic inflammatory stimulation of colonic mucosa. The method comprises a step of administering an effective amount of the amniotic fluid of the present invention or a pharmaceutical composition containing the amniotic fluid to a subject in need. The present invention further provides use of the amniotic fluid in the preparation of medicines for treating or preventing enteritis in a subject, for preventing intestinal cancer associated with chronic inflammatory stimulation of colonic mucosa in a subject, or for activating Nrf2 and inhibiting NF-κB to alleviate inflammation and oxidative damage caused by destruction of an intestinal mucosal barrier in a subject, and the amniotic fluid or a pharmaceutical composition thereof described herein for treating or preventing enteritis, preventing intestinal cancer associated with chronic inflammatory stimulation of colonic mucosa, or for activating Nrf2 and inhibiting NF-κB to alleviate inflammation and oxidative damage caused by destruction of an intestinal mucosal barrier in a subject.

In some embodiments, the present invention further provides a method for (1) retarding or preventing colon shortening in a subject, and/or (2) inhibiting splenomegaly in a subject, and/or (3) alleviating the progression of colitis, and/or (4) mitigating the symptoms (for example, colon mucosal damage, loss of tissue structure, epithelial erosion, reduction in the number of glands, and inflammatory cell infiltration, etc.) of acute enteritis (for example, acute UC) in a subject, and/or (5) increasing the abundance of goblet cells and improving goblet cell morphology in a subject to produce more mucus, and/or (6) downregulating the expressions of NF-κB p65, PIκB, IL-6 and TNF-α and upregulating the expressions of Occludin, ZO-1, Nrf2 and HO-1 in a subject. The method comprises a step of administering an effective amount of the amniotic fluid of the present invention or a pharmaceutical composition containing the amniotic fluid to a subject in need. Preferably, the subject is a patient with colitis.

In the present invention, an effective amount refers to a dose administered to a subject to achieve the treatment, prevention, alleviation and/or relief of a disease or a disorder. A therapeutically effective dose can be determined according to the factors such as a patient's age, sex, disorder and severity, and other physical conditions of the patient. In the present invention, a subject or a patient generally refers to a mammal, especially a human. In the present invention, the treatment and prevention have meanings well known in the art.

The amniotic fluid can be administered directly to a subject in need or can be used in the method and uses described herein. The mode of administration may be parenteral, for example, intravenous administration. In some embodiments, a therapeutically effective dose of amniotic fluid can be mixed with an appropriate amount of physiological saline for injection, water for injection, or glucose injection, and then administered, for example, by intravenous infusion. Preferably, a pharmaceutical composition administered may contain 5-40% (v/v) or 10%-35% (v/v), preferably 15-30% (v/v) of the amniotic fluid.

In general, a pharmaceutical composition containing the amniotic fluid also contains pharmaceutically acceptable excipients. In the present invention, "pharmaceutically acceptable excipients" refer to carriers, diluents and/or excipients that are pharmacologically and/or physiologically compatible with a subject and an active ingredient, including but not limited to antibiotics, humectants, pH regulators, surfactants, carbohydrates, adjuvants, antioxidants, chelating agents, ionic strength enhancers, preservatives, carriers, flow aids, sweeteners, dyes/colorants, flavoring agents, wetting agents, dispersing agents, suspending agents, stabilizers, isotonic agents, solvents or emulsifiers. In some embodiments, pharmaceutically acceptable excipients may include one or more inactive ingredients, including but not limited to stabilizers, preservatives, additives, adjuvants, enteric-coated agents, or other appropriate inactive ingredients used in combination with pharmaceutically effective compounds. The dosage and frequency of administration can be determined by medical staff according to the patient's specific condition, age and sex, etc. Generally, for the treatment of a particular disease, a therapeutically effective dose refers to an amount sufficient to ameliorate or alleviate the symptoms associated with the disease in a manner. Such an amount may be administered as a single dose or administered according to an effective treatment regimen. Doses may be administered to cure a disease, but are usually administered to ameliorate the symptoms of a disease. Repeated dosing is generally required to achieve the desired improvement of symptoms. For example, the dosage for human can usually be 1-200 mL/time, and can be administered by injection daily or weekly. In some embodiments, the frequency of administration may be multiple times per day, twice per day, once every two days, every three days, every four days, every five days, or every six days, or once every half a month or once a month.

The present invention also provides a pharmaceutical composition, which contains the amniotic fluid described herein, especially the amniotic fluid from poultry eggs, more preferably the amniotic fluid form chicken eggs with an embryonic age of 5-12 days, still more preferably 6-11 days, still more preferably 6-9 days and still more preferably 7-8 days. The pharmaceutical composition may be amniotic fluid or lyophilized reagent thereof frozen and stored below -60°C, for example, lyophilized amniotic fluid. The pharmaceutical composition may also contain other pharmaceutically acceptable carriers or excipients, for example, physiological saline for injection, water for injection, or glucose injection, etc. Preferably, the pharmaceutical composition contains 5-40% (v/v) or 10%-35%, preferably 15-30% of amniotic fluid.

The present invention will be illustrated below in combination with specific embodiments. It should be understood that these embodiments are illustrative only and are not intended to limit the scope of the present invention. Unless otherwise stated, methods, reagents and instruments used in the embodiments are conventional methods, reagents and instruments in the art.

### Materials and methods

### Preparation of ceAF

Fertilized eggs were incubated at 38±1°C and 50% relative humidity, and collected from chicken eggs at 6 to 8 days. Samples were centrifuged at 2500 g for 20 min, then the supernatant was filtered with a 0.22 µm sterilization device and stored at -80°C.

### Cell culture

Experimental cells were RAW264.7 mouse macrophage cell line from the National Collection of Authenticated Cell Cultures (NCACC). Cells were cultured in DMEM high-glucose medium containing 10% fetal bovine serum (Gibco, USA), 100 U/mL penicillin and 100 mg/mL streptomycin (Gibco, USA) in an incubator at 37°C and 5% CO₂. Nrf2 inhibitor ML385 was purchased from Selleckchem. Lipopolysaccharide (LPS) was purchased from Sigma. Cells were seeded in a 6-well plate at a density of 5 × 10⁵/mL, then ML385 dissolved in dimethyl sulfoxide (25 µM) was added and incubated at 37°C for 12 h, then 2 µg of LPS (1 µg/mL) and 10% CEAF were added and incubated at 37°C for 12 h.

### Cell viability test

The cell viability test was conducted in a 96-well plate by CCK-8 assay. After starvation for 12 hours to synchronize the cell cycle, cells were treated with 0, 2%, 4%, 6%, 8%, and 10% ceAF for 24 h respectively, and then washed three times with phosphate-buffered saline (PBS). Then, the incomplete medium (100 µL) was mixed with CCK-8 solution (10 µL). The optical density (OD) was measured at a wavelength of 450 nm by an ELISA analyzer.

### Experimental animals and interventions

C57BL/6J mice (female, 7-8 weeks old) were purchased from the Model Animal Research Center of Nanjing University. The mice were placed in a specific pathogen-free (SPF) environment with an ambient temperature of 22±1°C, a relative humidity of 50±1%, and a light/dark cycle of 12h/12h; and they were randomized into 5 groups (n=10):
(1) Control group: Animals were free to access to water and foods, and given distilled water by gavage;
(2) Colitis group (DSS, dextran sulfate sodium): Animals were given 3% DSS aqueous solution instead of distilled water by gavage;
(3) Low-dose ceAF treatment group (DSS+5%ceAF): Animals drank 3% DSS aqueous solution and were given 5%ceAF by gavage;
(4) High-dose ceAF treatment group (DSS+ ceAF): Animals drank 3% DSS aqueous solution and were given 10%ceAF by gavage;
(5) Large-dose ceAF group (distilled water + 10% ceAF): Animals were free to access to water and foods, and given 10% ceAF by gavage.

Experimental colitis was induced in mice by drinking water with 3% DSS (w/v) for 7 consecutive days. Starting from the day of DSS administration, mice were treated with ceAF by gastric intubation for 12 consecutive days. The mice in the normal control group only drank sterile water. The mice's behaviors, body weights, food intake and stool state were measured or observed every day. Two weeks later, the mice were sacrificed, and specimens were collected and stored at - 80°C. Fluorescent staining of the colon was performed.

### Assessment of body weights, disease activity index (DAI) score, visual assessment of ulcers, and colon length

During the experiment, changes in body weights, stool form and occult blood in stools were recorded, and the disease activity index (DAI) score was calculated. The DAI score can be used to assess the severity of disease according to the extent of weight loss, stool consistency and the amount of blood in the stool. The DAI score was calculated according to the formula: DAI= (weight loss score + stool form score + stool blood score)/3. The weight loss score was based on the percentage of weight loss: 0 (weigh loss by 0%), 1 (weight loss by 1-5%), 2 (weight loss by 5-10%), 3 (weight loss by 10-20%), 4 (weight loss by >20%. In terms of stool consistency, the scores were given as follows: normal particles (0), loose stools (1), semi-formed stools (2), liquid stools (3) and diarrhea (4). In terms of blood amount, the scores were given as follows: 0 (no blood), 1 (tract amount), 2 (mild occult blood), 3 (obvious occult blood), and 4 (major bleeding). Then the subscores were summed and divided by 3 to obtain the DAI score (0-4). The colon was analyzed under a stereomicroscope, and any obvious damage was scored from 0 to 5 according to the scoring criteria proposed by the previously described well-established scoring method, which considered the area of inflammation and the presence or absence of ulcers. The visual evaluation criteria of ulcers: 0 (no ulcers and no inflammation); 1 (no ulcers and local congestion); 2 (ulcers without congestion); 3 (ulcers and inflammation in only one site); 4 (ulcers and inflammation at two or more sites); 5 (range of ulcers exceeding 2 cm). The mice were sacrificed by cervical dislocation, the colon from the cecum to the anus was cut, and its length was measured.

### Hematoxylin-eosin (HE) and periodic acid-Schiff (PAS) staining

For histopathological evaluation, the distal colon part was subjected to 10% formalin fixation and paraffin embedding, and HE staining. The severity of DSS-induced tissue damage was graded by a histological scoring system. Based on the tissue damage percentage, the scoring was as follows: 0 (no tissue damage), 1 (1-25% of tissue damage), 2 (26-50% of tissue damage), 3 (51-75% of tissue damage), 4 (76-100% of tissue damage). Based on the conditions of tissue damage, the scoring was as follows: 1 (mucosa), 2 (mucosa and submucosa), and 3 (beyond the submucosa). Based on the extent of crypt damage, the scoring was as follows: 1 (damage to 1/3 of the bottom part), 2 (damage to 2/3 of the bottom part), 3 (only the epithelial surface remaining intact), and 4 (loss of the entire crypt and epithelium). Based on the severity of inflammation, the scoring was as follows: 1 (mild), 2 (moderate), and 3 (severe). At the same time, PAS staining was performed to determine the mucus-secreting cells.

### Western blot analysis

The colon tissues were extracted with RIPA buffer and centrifuged (5000×g, 10 min) to obtain protein. The protein concentration was measured with BCA protein assay kit. Equal amounts of protein (30 µg) were separated by 10% SDS-polyacrylamide gel electrophoresis, transferred to nitrocellulose membrane, and blocked in 5% BSA buffer for 90 min. After incubation with TLR4, pIR4, IL6, NF64 p65, TNF-α, Keap1, ZO-1, Occludin, HO-1 and Nrf2 primary antibodies (1:1000 dilution) overnight at 4°C, the membrane was rinsed with TBS-T (pH7. 4) three times for 6 minutes, then incubated with HRP-labeled anti-goat secondary antibody (1:40000 dilution) for 90 minutes. After washing with TBS-T three times for 6 minutes, all membranes were visualized with immobilon western chemiluminescent HRP substrate. The expression level of each protein was normalized to the internal reference GAPDH.

### Cytokine levels and assessment of antioxidant activity

The levels of TNF-α and IL-6 in serum were detected with commercial ELISA kit. In addition, after the colon was resected and homogenized at 4°C, the total protein content was evaluated using the BCA protein assay kit. The activities of GSH-Px, MDA, MPO and SOD in colon tissues were determined by chromatography.

### RNA extraction and mRNA expression analysis

Total RNA was extracted according to the instructions of the FastPure Cell/Tissue Total RNA Isolation Kit. In order to prepare cDNA, RNA samples (500 ng) were subjected to cDNA synthesis using PrimeScript Real-Time Master Mix. The real-time PCR assay of samples was performed on the real-time fluorescence quantitative PCR system QuantStudio 5 using the Applied Biosystems Power SYBR Green PCR Master Mix reagent. The relative expression of each target gene was normalized to the GAPDH. Table 1 lists the primer sequences of the target genes (SEQ ID NO: 1-16). The critical threshold cycle (CT) value and relative mRNA concentration (E = 2^{-ΔΔCt}) were measure for each reaction.

**Table 1: RT-qPCR primer sequences**

| Gene | Forward primer | Reverse primer |
|---|---|---|
| *TNF-α* | TCTCCAGCCACCAGCCCTCTAA | TGGCCATGGTAGGAGAAACAGG |
| *IL-6* | ACAAAGCCAGAGTCCTTCAGAG | GGCAGAGGGGTTGACTT |
| *IL-1β* | TCCAGGATGAGGACATGAGCA | GAACGTCACACACCAGCAGGT |
| *Nrf2* | AACAACGCCCTAAAGCA | TGGATTCACATAGGAGC |
| *HO-1* | CACGTATACCCGCTACCT | CCAGTTTCATTCGAGCA |
| *Occludin* | GGCGGATATACAGACCCAAGAG | GATAATCATGAACCCCAGGACAAT |
| *ZO-1* | GAGCGGGCTACCTTACTGAAC | GTCATCTCTTTCCGAGGCATTAG |
| *GAPDH* | CCAGTATGACTCCACTCACG | GACTCCACGACATACTCAGC |

### Immunofluorescence (IF) detection

Colon tissues were fixed using 4% paraformaldehyde and then embedded in paraffin. Tissue slides were deparaffinized using xylene, then polarized by reducing the alcohol concentration and washed with deionized water. Bovine serum albumin (1%) was used to block for 1 h; then, the slides were incubated with primary antibodies (4°C) overnight. Afterwards, Alexa Flour 488 or 594 labeled secondary antibodies were used for staining. After completion of the secondary antibody incubation, the cell nuclei are stained with DAPI solution, and the entire procedure was performed under the condition away from light. Images were captured using a Leica DMIRE2 confocal laser scanning microscope.

### Statistical Analysis

The statistical analysis of all test results was performed by GraphPad Prism v6.0, and data were expressed as mean ± standard deviation (SD). The two-way ANOVA and least significant difference method were used to compare differences between groups. The P values were set at <0.05, <0.01 and <0.001 at the significance level.

### Results

### 1. The effect of ceAF on alleviating the symptoms in mice with DSS-induced colitis

To evaluate the potential protective effect of ceAF in mice with DSS-induced colitis, mice were treated according to the above method to establish a colitis model (FIG.1, A). Weight loss, stool consistency, and bleeding are notable symptoms of DSS-induced colitis. The weight loss, DAI, colon length, and intestinal damage were observed. Five days after DSS intake, the body weights of the mice were significantly reduced, while ceAF alleviated this trend (FIG.1, B). Colon length is another reproducible indirect indicator of enteritis severity. Compared with the control group, the colon lengths of mice given with DSS were significantly shortened. Surprisingly, supplementation with ceAF significantly reversed colon shortening in mice exposed to DSS (FIG.1, C). Splenomegaly accompanied by inflammatory changes caused by DSS was also observed. However, ceAF treatment could inhibit DSS-induced splenomegaly (FIG.1, D). Continuous use of ceAF could alleviate the stool form, occult blood, and even purulent blood-like fecal changes in mice in the colitis group. Mice exposed to DSS had increased DAI scores (an index of the severity of colon inflammation), compared with those that drank water only. After administration of ceAF, these symptoms were significantly improved (FIG.1, E). Overall, these observation results indicated that the ceAF intake effectively relieved the progression of colitis, and a high concentration (10% ceAF) had a better therapeutic effect than a low concentration (5% ceAF).

### 2. ceAF improved histological parameters of mice with DSS-induced colitis

The histopathological analysis further confirmed the effect of ceAF on DSS-induced colitis. The ulcerative colitis (acute phase) exhibited a number of histological features, including mucosal erosion, crypt shortening, edema, and inflammatory cell infiltration of the mucosa and lamina propria. Data from H&E stained sections showed that the morphological structure of colon tissues was significantly different. Compared with the control group, mice in the DSS group showed severe damage to the colon mucosa, loss of tissue structure, severe epithelial erosion, significant reduction in the number of glands, and significant inflammatory cell infiltration. In contrast, acute UC inflammatory symptoms were alleviated in mice treated with 5% or 10% ceAF (FIG.2, A). Administration of ceAF also decreased the histological score of DSS-induced colitis (FIG.2, C), indicating that ceAF had a significant protective effect against inflammation-induced intestinal damage in mice.

PAS staining was used to assess the abundance and integrity of goblet cells in the colon's mucus layer, the primary barrier protecting the intestine. DSS significantly reduced the abundance of goblet cells in mice in the colitis group compared with that in the control group, and there was no mucus layer in the area where inflammation occurred (FIG.2, B). Surprisingly, the mice treated with ceAF showed significant increases in goblet cell abundance and morphological improvements, and the goblet cells produced more mucus (FIG.2, B). These recoveries may be attributed to the preservation of epithelial goblet cells, which produce various mucins and are primarily responsible for reducing tissue damage. Furthermore, higher concentrations of ceAF appeared to show better therapeutic effects.

### 3. Recovery of DSS-induced inflammatory response and colonic barrier dysfunction after oral administration of ceAF

The Nrf2 pathway plays an important role in the cellular defense system by regulating a series of detoxifying enzymes and antioxidant proteins, including HO-1, SOD and GSH-Px. This regulation leads to a decreased risk of intestinal inflammation and oxidative stress. HO-1 is the rate-limiting enzyme in heme catabolism and has an endogenous defense mechanism. Activation of Nrf2 positively regulates HO-1 transcription and is critical for reducing the risk of gastrointestinal inflammation and oxidative stress. NF-κB is a redox-sensitive transcription factor that is very important for inflammation, innate immunity, and maintaining tissue integrity. It regulates the expression of a variety of pro-inflammatory factors, such as IL-6, IL-1β, tumor necrosis factor-α, cyclooxygenase-2 and chemokines. These cytokines ultimately induce local inflammation and immune dysfunction, and these changes trigger a positive feedback loop, leading to the development of inflammation and intestinal mucosal damage.

In order to explore the possible mechanisms of the above therapeutic effects, Western blot and real-time fluorescence quantitative PCR methods were used to detect the protein and mRNA expression levels of cytokines in the intestinal tissues of mice after administration of ceAF. Compared with the control group, the expression levels of NF-κB p65, PIκB, IL-6, and TNF-α were significantly increased in DSS-induced colitis mice, while the expression levels of Nrf2 and HO-1 were significantly decreased (FIG.3, A and B). Interestingly, the expression levels of these typical inflammation and oxidative stress indexes were significantly down-regulated after treatment with ceAF (FIG.3, A and B). Consistent with protein expression, ceAF treatment significantly suppressed these typical indexes at the mRNA level (FIG.3, C-G). Compared with the control group, the protein expression levels of ZO1 and occludin were significantly decreased in the DSS-treated group, indicating that the tight junction (TJ) structure was destroyed. In contrast, treatment with ceAF reversed the expression levels of these proteins completely (FIG.3, A, H, and I). In summary, the protective efficacy of 10% ceAF was better than that of 5% ceAF. Immunofluorescence staining was used to detect M1 macrophages in colon tissue treated with 10% ceAF. The results showed that the treatment with 10% ceAF significantly reduced the number of M1 macrophages (FIG.3, J and K).

### 4. ceAF regulated enzymes and inflammatory cytokines involved in the oxidative stress response in DSS-induced experimental colitis

Colitis (UC) is characterized by the persistent expression of various inflammatory cytokines (such as IL-1β, IL6, and TNF-α) and the accumulation of oxidative stress. The use of antioxidant enzymes can effectively resist the inflammation-related diseases by targeting GSH-Px, MDA, MPO, and SOD.

Compared with the control group, DSS induced a significant decrease in SOD and GSH-Px activities in colon tissues (FIG.4, A and B). In contrast, MDA and MPO activities were activated after DSS treatment, but intragastric intubation with 10% ceAF could significantly reverse the DSS-mediated changes (FIG.4, C and D). Furthermore, the IL-6 and TNF-α levels in serum were significantly upregulated in colitis mice compared with control mice. However, this upregulation was significantly inhibited by 10% ceAF (FIG.4, E and F). Results of immunofluorescence analysis (IF) (FIG.4, G and H) were consistent with those of Western blot (FIG.3, A). Our studies showed that ceAF might directly inhibit the upregulation of pro-inflammatory cytokines and oxidative stress by activating Nrf2 and inhibiting the NF-κB signaling pathway.

### 5. ceAF alleviated inflammation and oxidative stress in lipopolysaccharide (LPS)-stimulated RAW 264.7 cells via NF-κB and NRF2 signaling pathways

Nrf2 deficiency can exacerbate enteritis in animal models of various diseases (such as emphysema, pleurisy, and sepsis), indicating that it plays a crucial role in mediating inflammatory responses and oxidative stress. Biocompatibility is an important consideration when evaluating the efficacy of anti-inflammatory and antioxidant drugs on target cells. Therefore, the *in vitro* cytotoxicity of ceAF was determined by measuring the viability of treated cells.

Our studies showed that ceAF exhibited better biocompatibility at concentrations lower than 10% (FIG.5, A). However, when the ceAF concentration exceeded 40%, the cell survival rate decreased significantly (FIG.5, A). Therefore, the subsequent protein expression related to inflammation and oxidative stress was studied in lipopolysaccharide-stimulated RAW 264.7 cells treated with or without 10% ceAF. Lipopolysaccharide significantly upregulated the expression levels of TLR4, PIκB, NPκB p65, IL 6, TNF-α, and Keap1, and decreased the expression levels of Nrf2 and HO1 in RAW 264.7 cells (FIG.5, B and C), indicating that lipopolysaccharide significantly activated the signaling pathways related to inflammation and oxidative stress. Interestingly, the expression of these proteins was significantly reversed after treatment with 10% ceAF. To further determine the underlying mechanism of the antagonistic effect of ceAF on endotoxin-stimulated RAW 264.7 cells, an Nrf2 inhibitor (ML385) was used to conduct experiments. It was found that ML385 effectively inhibited the activity of Nrf2, and these inflammatory and oxidative stress effects of ceAF on endotoxin-stimulated RAW 264.7 cells could be counteracted by pretreatment with ML385. Therefore, it was demonstrated that ceAF might directly inhibit the upregulation of pro-inflammatory cytokines and oxidative stress by activating Nrf2 and inhibiting NF-κB.

### Conclusion

In conclusion, the data have shown that ceAF can reduce the severity of DSS (dextran sulfate sodium)-induced colitis in terms of macroscopic and histological parameters. In addition, ceAF also reduces inflammation and oxidative damage caused by intestinal mucosal barrier damage by regulating Nrf2 and NF-κB signaling pathways.

## Claims

1. Use of amniotic fluid in the preparation of medicines for treating or preventing enteritis in a subject; wherein, the amniotic fluid is from chicken eggs with an embryonic age of 5-12 days, or from eggs of poultry other than chickens whose development stage corresponds to the development stage of chicken eggs of the embryonic age, or from embryos of rodents with an embryonic age of 8-14 days, or from embryos of non-human mammals other than rodents whose development stage corresponds to the development stage of rodents with an embryonic age of 8-14 days.

2. The use of claim 1, wherein the amniotic fluid is from chicken eggs with an embryonic age of 6-11 days.

3. The use of claim 1, wherein the amniotic fluid is from chicken eggs with an embryonic age of 7-9 days, more preferably chicken eggs with an embryonic age of 7-8 days.

4. The use of claims 1 to 3, wherein the enteritis is enteritis of small intestine and colitis.

5. The use of claims 1 to 3, wherein the enteritis is chronic enteritis, including chronic bacillary dysentery, chronic amoebic dysentery, schistosomiasis, non-specific ulcerative colitis and regional enteritis.

6. The use of claims 1 to 3, wherein the enteritis is inflammatory bowel disease, including ulcerative colitis and Crohn's disease.

7. Use of amniotic fluid in the preparation of medicines for preventing intestinal cancer in a subject; wherein, the amniotic fluid is from chicken eggs with an embryonic age of 5-12 days, or from eggs of poultry other than chickens whose development stage corresponds to the development stage of chicken eggs of the embryonic age, or from embryos of rodents with an embryonic age of 8-14 days, or from embryos of non-human mammals other than rodents whose development stage corresponds to the development stage of rodents with an embryonic age of 8-14 days.

8. The use of claim 7, wherein the amniotic fluid is from chicken eggs with an embryonic age of 6-11 days, preferably chicken eggs with an embryonic age of 7-9 days, more preferably chicken eggs with an embryonic age of 7-8 days.

9. Use of amniotic fluid in the preparation of medicines for activating Nrf2 and inhibiting NF-κB to alleviate inflammation and oxidative damage caused by destruction of an intestinal mucosal barrier in a subject; wherein the amniotic fluid is from chicken eggs with an embryonic age of 5-12 days, or from eggs of poultry other than chickens whose development stage corresponds to the development stage of chicken eggs of the embryonic age, or from embryos of rodents with an embryonic age of 8-14 days, or from embryos of non-human mammals other than rodents whose development stage corresponds to the development stage of rodents with an embryonic age of 8-14 days.

10. The use of claim 9, wherein the amniotic fluid is from chicken eggs with an embryonic age of 6-11 days, preferably chicken eggs with an embryonic age of 7-9 days, more preferably chicken eggs with an embryonic age of 7-8 days.

11. Use of amniotic fluid in the preparation of pharmaceutical compositions for one or more of the following uses:
(1) retarding or preventing colon shortening in a subject;
(2) inhibiting splenomegaly in a subject;
(3) alleviating the progression of colitis;
(4) mitigating the acute enteritis in a subject;
(5) increasing the abundance of goblet cells and improving goblet cell morphology in a subject to produce more mucus; and
(6) downregulating the expressions of NF-κB p65, PIκB, IL-6 and TNF-α and upregulating the expressions of Occludin, ZO-1, Nrf2 and HO-1 in a subject;
wherein, the amniotic fluid is from chicken eggs with an embryonic age of 5-12 days, or from eggs of poultry other than chickens whose development stage corresponds to the development stage of chicken eggs of the embryonic age, or from embryos of rodents with an embryonic age of 8-14 days, or from embryos of non-human mammals other than rodents whose development stage corresponds to the development stage of rodents with an embryonic age of 8-14 days.

12. The use of claim 11, wherein the amniotic fluid is from chicken eggs with an embryonic age of 6-11 days.

13. The use of claim 12, wherein the amniotic fluid is from chicken eggs with an embryonic age of 7-9 days, more preferably chicken eggs with an embryonic age of 7-8 days.

14. The use of any one of claims 11 to 13, wherein symptoms of the acute enteritis are selected from one or more of colon mucosal damage, loss of tissue structure, epithelial erosion, reduction in the number of glands, and inflammatory cell infiltration.

15. The use of any one of claims 11 to 14, wherein the subject is a patient with enteritis.
